# EUROPEAN PATENT SPECIFICATION

(11) **EP 1 942 799 B1**
(45) Date of publication and mention of the grant of the patent: **19.08.2015**
(21) Application number: 06802831.5
(22) Date of filing: 01.09.2006
(51) Int. Cl.: A61B 5/0408, A61B 5/0428, A41D 13/12, A61B 5/0476, A61B 5/0488

(54) **PHYSIOLOGICAL MONITORING WEARABLE HAVING THREE ELECTRODES**
TRAGBARES PHYSIOLOGISCHES ÜBERWACHUNGSGERÄT MIT DREI ELEKTRODEN
SYSTÈME PORTABLE DE SURVEILLANCE PHYSIOLOGIQUE COMPORTANT TROIS ÉLECTRODES

(30) Priority: 30.09.2005 US 163017
(43) Date of publication of application: 16.07.2008
(73) Proprietor: Textronics, Inc., Wilmington, DE 19805 (US)
(72) Inventor: WIJISIRIWARDANA, Ravindra, Wilmington, DE 19807 (US)
(74) Representative: Wegner, Hans
(86) International application number: PCT/US2006/034262
(87) International publication number: WO 2007/040878

(56) References cited:
- WO-A-2004/097089
- WO-A2-01/02052
- US-A1- 2005 034 485
- US-B1- 6 496 721

## Description

### FIELD OF THE INVENTION

The present description relates to a wearable item in the form of, for example, a garment, band, or patch worn on or about the body, including, in part, at least three fabric electrodes, such as metallized fabric electrodes. More particularly, the description relates to a monitoring apparatus and method to receive signals correlated with at least one physiological event or physiological characteristic of a wearer. Specifically, the description provides a wearable item comprising at least three conductive electrodes of, for example, stretch-recovery electrically conductive yarns embedded with non-conductive stretch-recovery yarns, which make up the remaining portion of the wearable item. This wearable item may further include means for using the three electrodes to monitor at least one biophysical event or biophysical characteristic of the wearer. Specifically, at least the wearer's electrical characteristics and heart rate can be monitored with improved resolution and stability.

### BACKGROUND OF THE INVENTION

Conductive electrodes and an electrode system incorporated into a wearable item, such as a garment, have been disclosed. For example, wearable conductive sensors having two electrodes for sensing or otherwise reporting the heart rate (the pulse) of the wearer are disposed in patent document WO 02/071935, assigned to RTO Holding OY.

U.S. 2006/0211934 (Pat. Appln. No. 11/082,240) also discloses garment and wearable systems having at least one conductive electrode. The garment and wearable systems disclosed in this application include a fabric portion having stretch-recovery non-conductive yarns and a stretch-recovery electrically conductive region of electrically conductive yarn filaments. Such conductive electrode system(s) provide first and second fabric portions that include electrically conductive regions. The electrically conductive regions are disposed in a partially overlapping relationship, allowing for a region of partial physical contact that can result in electrical conduction between the electrically conductive regions and skin. At least one of the electrically conductive regions may include a float yarn. In addition, at least one of the electrically conductive regions can be made up of an elastified electrically conductive yarn and/or an elastic yarn at least partially plated with a conductive yarn. In one embodiment, the electrically conductive regions include a fabric having a textured or ribbed construction. Such conductive electrodes can be connected to a measuring device to monitor physiological events or biophysical signals of a wearer of a garment incorporating the electrodes. For example, the conductive electrodes can be used to facilitate monitoring a wearer's electrical activity to derive heart rate. For instance, a "sports bra" for heart rate monitoring systems employs two integrated fabric electrodes. This two-electrode construction may be accompanied with a significant degree of noise in the detected heart signal. In this regard, it is believed that motion of the sports bra wearer may contribute to this electric al noise, and that a design having more than two electrodes may be advantageous to reduce electrical noise.

Electrocardiogram or ECG is the measurement of the electrical signals or characteristics of the human heart (and/or mammalian and other hearts). In conventional ECG measurement, skin-surface electrodes are placed on four limbs or the chest of the subject to be measured (Bioimpedance & Bioelectricity Basics, S. Gimnes and O.G. Martinsen; Academic Press, 2000, pages 268-269). The four electrodes used in such conventional ECG practice typically employ bipolar voltage recording of three potential differences. A fourth electrode is attached to the right leg of the subject serves as the ground or reference. According to S. Gimnes et al., in the work cited above, the signal amplitude of these three potential differences is typically about one millivolt (mV) and the bandwidths measured are in the range of about 0.05 to about 100 Hertz (Hz) with DC filtering.

A three textile electrode-based arm and chest band for ECG and heart rate monitoring was disclosed in a paper entitled "Fiber-Meshed Transducers Based Real Time Wearable Physiological Information Monitoring System" by Wijesiriwardana et al. in the Proceedings of the Eighth International Symposium on Wearable Computers (ISWC 2004) sponsored by the IEEE Computer Society ("Wijesiriwardana"). Wijesiriwardana disclosed an arrangement of three bands, with each band including one sewn on electro-conductive fabric structure on a non-conductive elastomeric structure. One band was disclosed as encircling the chest of the subject to be monitored, and the other two bands were disclosed to be worn on each upper arm. These three electrodes were connected to a preamplifier, where one arm electrode functioned as a reference electrode and the other two electrodes functioned as.differential inputs to the preamplifier electronics. According to Wijesiriwardana, pre-amplifier electronics were designed with AC coupled signal and high pass "RC passive" filtering to overcome the high fluctuations in the observed signal and the very low signal level of the ECG potential. Fundamentally, the three electrode configuration of Wijesiriwardana employs "cut and sew" electrode patches of electro-conductive fabrics sewn to a substrate fabric of elastic material. The three electrode system of Wijesiriwardana is not a unitary design, meaning that the three electrodes were placed on separately worn bands of the subject being measured.

Document US 2005/0034485 A1 relates to a garment for the medical monitoring of a patient. A flexible, elastic garment comprising biomedical sensors, means for contactless transmission of power and data between the sensors and means for receiving and processing the data, characterized in that the sensors, and the means for distributing the electrical power and/or transporting the data from the sensors in the garment are formed by elastic conducting yarns integrated into the fabric of the garment. US 2005/0034485 discloses the features as defined in the preamble of claim 1.

Further, US 6,496,721 B1 relates to a voltage sensing system includes input impedance balancing for electrocardiogram (ECG) sensing or other applications, providing immunity to common-mode noise signals while capable of use with two electrodes. In one embodiment, an the ECG detector includes a right leg cable RL (used for attaching a third electrode to the right leg of a patient) used in a feedback arrangement coupled to a differential amplification circuit.

There exists a need in the art for an ECG and heart rate monitoring system comprising a single wearable unit with electronics capable of collecting and amplifying an ECG signal while having the capability to simultaneously reject electrical noise in the low level ECG and heart rate signals. Such a system for monitoring ECG and heart rate could be conveniently constructed for the wearer as, for example, a whole garment, e.g., a bra, especially a "sports bra", or shirt or vest singlet suitable for both sexes.

### SUMMARY OF THE INVENTION

The present invention provides a wearable system for monitoring at least one physiological event or physiological characteristic of a wearer as defined in claim 1. The wearable system includes a wearable item comprising: (i) at least one non-electrically conductive yarn; and (ii) three conductive electrodes. The wearable system further includes at least one means for conducting from the detected electrical signals associated with the at least one physiological event or physiological characteristic of the wearer.

In at least one embodiment, at least one of the conductive electrodes may include a conductive yarn having stretch-and-recovery properties.

The wearable system includes means for conducting electrical signals electrically linked to at least one means for signal pre-processing, at least one physiological event or physiological characteristic.

Physiological events or characteristics that can be monitored by the invention, while not limited, can include, for example, ECG or heart rate, breathing rate, electroencephalogram (EEG); electromyogram (EMG), and Electro Gastrogram (EGG).

Wearables devices of the invention can, for example, be in the form of a garment such as a bra, a shirt, an undergarment, a vest, a bodysuit, a sock, a glove, a stocking, a belt, a band, a strap, or a jacket.

The present invention is defined by the appended claims that follow. The embodiments, examples, or aspects of the present description which do not fall within the scope of said claims are provided for illustrative purposes only and do not form part of the present invention.

### BRIEF DESCRIPTION OF THE DRAWINGS

FIG. 1 is a schematic representation of a garment of the prior art having two skin-contacting electrodes;
FIG. 2 is a schematic representation of a garment according to the invention having three skin-contacting electrodes;
FIG. 3 is an enlarged cross-sectional view of the garment taken along line 3-3 of FIG. 2;
FIG. 4 is a schematic representation of an electronic circuit diagram useful for the receiving and amplifying physiological signals from a wearable device having three-skin contacting electrodes according to the invention;
FIG. 5 is a block-diagram representation of the amplifier circuit of FIG. 4;
FIG. 6 is a graphical representation of heart waveforms from a prior art heart rate monitoring belt or band having two skin contacting electrodes; and
FIG. 7 is a graphical representation of heart rate waveform from a heart rate monitoring belt or band according to this invention having three skin-contacting electrodes.

### DETAILED DESCRIPTION

The present description, in one embodiment, can provide an improved wearable or garment system for monitoring at least one physiological event or physiological characteristic of a wearer. The wearable or garment includes at least one substantially non-electrically conductive stretch-recovery yarn and at least three conductive electrodes of, for example, stretch-recovery electrically conductive yarns integrated with the wearable device. Also included in the wearable systems is at least one means for conducting electrical signals associated with at least one physiological event or physiological characteristic of the wearer from the garment to an external means. The external means can, for example, be used to signal pre-process, preamplify, amplify, process, display, filter, analyze, alarm and/or store electrical signals associated with at least one physiological event or physiological characteristic of the wearer.

As used herein, the term "wearable" refers to any article of manufacture designed to be worn on or borne by the body or any portion of the body of a wearer. When in the form of a garment, the wearable can, for example, be in the form of a bra, shirt (including, for example, a tank top), undergarment (such as an undershirt or underpants), vest, sock, sleeve, glove, stocking, bodysuit, or jacket. The term "wearable" encompasses not only garments, but also bands, straps, belts, hats, patches, etc. When in the form of a band, the wearable can, for example, be in the form of a torso band, waist band, arm band, leg band, neck band, or wrist band.

As used herein, the terms "physiological event" or "physiological characteristic" refer to measurable parameters that relate to a physiological condition of a subject. Examples of physiological events and physiological characteristics include, but are not limited to signals that can identify ECG and heart rate, breathing rate, electroencephalogram (EEG), electromyogram (EMG), and Electro Gastrogram (EGG), as examples.

As used herein, the term "substantially non-electrically conductive stretch-recovery yarn" refers a continuous yarn made from one or more continuous filaments each of which is made from a substantially electrically insulating elastomeric material which provides for an elongation before the elastic limit is reached of up to 800% of the gauge length and a subsequent retraction to the original gauge length with no substantial set.

As used herein, "strenuous activity" can be defined as activity in which a wearer perspires, such that the skin becomes moist or wet.

As used herein, "non-strenuous activity" can be defined as activity in which the skin is essentially dry.

As used herein, "high-movement activity" can be defined as activity in which the part of the body in contact with at least one conductive electrode experiences a high degree of relative movement or displacement.

As used herein, "low-movement activity" can be defined as activity in which the part of the body in contact with the at least one conductive electrode experiences a low degree of relative movement or displacement.

Examples of high-movement strenuous activity include running, jogging, hiking, rowing, aerobic exercise or dancing, and competitive sports (basketball, football, racquetball, tennis, etc.). Examples of high-movement non-strenuous activity include walking, riding horses, sky diving, hang gliding, bungee jumping, riding roller coasters, trampoline jumping and golfing. Examples of low-movement non-strenuous activity include watching television, sitting in front of a computer, and resting in a stationary position (such as a patient in a hospital bed). An example of low-movement strenuous activity would be riding a stationary bike or wheelchair (where the electrodes contact the body above the waist).

The wearable system of the description can be used to monitor at least one physiological event or characteristic of a human wearer. It can also be used to monitor at least one physiological event or characteristic of an animal wearer, such as a horse, or a non-human primate (NHP), such as a chimpanzee or gorilla.

The wearable system disclosed herein can be adapted for measurement of at least the ECG and heart rate of a wearer obtained using at least three conductive electrodes. The wearable system disclosed herein can also be adapted to monitor the measurement of other physiological events or physiological characteristics of a wearer such as, breathing rate, electroencephalogram (EEG), electromyogram (EMG), and Electro Gastrogram (EGG). For example, in the three electrode embodiment, three electrodes are configured to contact the body corpus of the wearer securely via the stretch-recovery properties of the bulk of the wearable item.

The bulk of the wearable item can include any substantially non-electrically conductive stretch-recovery yarn in addition to other materials commonly used in fabric and textile applications. For example, the bulk of the wearable item can be made of elastomeric yarns (such as spandex) and comfort yarns (such as nylon, polyester, and/or cotton). In one embodiment, the non-conductive zones of the wearable can include a portion of LYCRA® brand (from INVISTA S. a r. I.) spandex (an example of such a wearable item is a sports bra). Such spandex may be covered with or combined with, for example: (i) nylon yarns or (ii) polyester yarns or polyester yarns combined with natural fiber yarns like cotton. In addition, the bulk of the wearable item can further include one or more layers of fabric or material.

Methods by which the conductive electrodes can be incorporated within the bulk of the wearable are not limited and include being woven or knitted into and/or on the wearable item, being printed on the wearable item, being heat-transferred on the wearable item, being glued, laminated or sandwiched on or between layers of the wearable item, and being mechanically fastened on the wearable item (by means of snaps, etc.). The conductive electrodes can be integrated into the structure of the wearable item by weaving or knitting methods. Methods for weaving or knitting conductive electrodes with bulk fabrics are disclosed, for example, in U.S. 2006/0211934 (Pat. Appln No. 11/082,240), filed March 16, 2005. Methods for laminating conductive elements between elastic layers of textile structures are disclosed in PCT Appln WO 2005/123377 (PCT/IB2005/001682), filed June 15, 2005..

The conductive electrode electrodes can be made from a variety of materials. For example, conductive electrodes can include materials such as metal wires, conductive fibers, conductive inks, conductive polymers, conductive yarns, and metals (such as in metal snaps or rivets).

Conductive yarns, useful in making conductive electrodes, can, for example, be metal-coated yarns, e.g., yarns coated with silver (Ag) or other suitable metals. Such conductive yarns can include yarns having intrinsic conductivity, such as (i) yarns having metal filaments or particles added to a synthetic polymer comprising the bulk of the yarn filaments, or (ii) intrinsically electrically conductive yarns, such as polyaniline; or (iii) a combination of the above. The conductive electrodes can exhibit stretch by using different types of knit constructions, such as a ribbed construction (including, for example, 1 x1 or 1 x3 ribbed knit constructions), as disclosed, for example, in U.S 2006/0211934 (Pat. Appln No. 11/082,240), filed March 16, 2005. In addition, the textile-electrodes may be knitted with stretch and recovery conductive yarns of the type disclosed in U.S. Published Pat. Appln No. 2004/0237494 A1. Such yarns include those in which an elastic material, such as spandex, is twisted or wrapped with a conductive material, such as a metal wire.

Further included in embodiments of the present description are means for conducting electrical signals associated with at least one physiological event or physiological characteristic of a wearer, which can, for example, allow such electrical signals to be transmitted to an external means for signal pre-processing, preamplifying, amplifying, processing, displaying, filtering, analyzing, alarming and/or storing such physiological event or characteristic. Such means for conducting electrical signals can include at least one conductive surface or region, which surface or region can include the conductive electrodes.

In one embodiment, at least one conductive surface or region is capable of having direct contact with both a wearer's skin and the outside surface of the wearable item. In another embodiment, at least one conductive surface or region is capable of having direct contact with a wearer's skin, where at least one interconnect device or material is capable of electrically linking at least one conductive surface or region to the outside surface of the wearable item via a conductive interconnect bridge. Such conductive interconnect bridge can, for example, include at least one: (a) mechanical fastening means (such as a snap); (b) conductive thread or wire; (c) touching interior conductive float (as disclosed in U.S. Pat. Appln No. 11/082,240); (d) metal grommet; (e) conductive glue or hot melt; and/or (f) fuzzy interior surface brush contact.

Configurations of the at least one conductive surface or region are not limited, and can include a configuration in which at least two conductive surfaces or regions are aligned horizontally or vertically relative to each other. For example, when the wearable is in the form of a band, strap or belt, conductive surfaces or regions can be aligned horizontally to each other along at least a lengthwise portion of the band, strap or belt. In another embodiment, three conductive surfaces or regions can be arranged in a triangular configuration, wherein one surface or region is above, below, to the left, or to the right of the other two surfaces or regions.

In order to achieve satisfactory noise suppression, the three conductive electrodes can be "balanced" meaning that, from an electrical impedance perspective, each of the electrodes are essentially electrically identical with regard to their interaction with the body. In other words, when the three conductive electrodes are "balanced" the electrode skin impedances of the three electrodes are essentially the same.

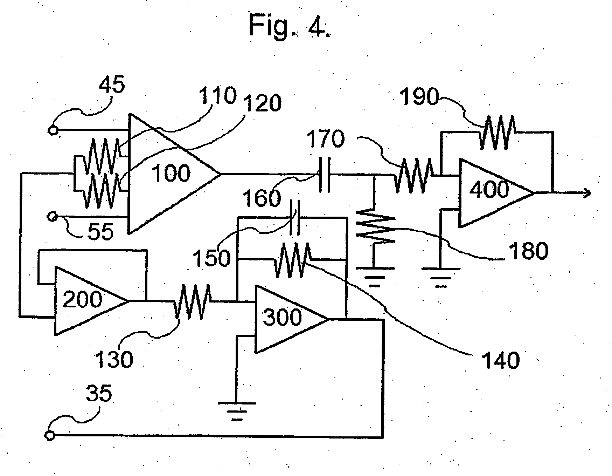

The present invention, in an embodiment, includes a tuned low-gain high input impedance first stage of amplification of the electrical signals fed to the pre-processing circuit from at least two electrodes. Tuned herein means to provide appropriate impedance matching from the electrodes to circuit input based upon the expected frequency range being amplified. Such tuning means are conventional in the art and generally involve a selection of the discrete electrical elements of the circuit represented by Fig. 4. One skilled in the art would know, a *priori,* how to select the values of resistance and capacitance to achieve tuning of the amplifier circuit and electrodes in combination with knowledge of the frequency of the electrical signals.

The present invention, in another embodiment, includes an improved wearable system, such as a garment system, further comprising at least one means for signal pre-processing, preamplifying, amplifying, processing, displaying, analyzing, filtering, alarming, or storing at least one physiological event or physiological characteristic, such as a wearer's ECG and heart rate. At least one physiological event or physiological characteristic can be transmitted to the at least one means for signal pre-processing, preamplifying, amplifying, processing, displaying, analyzing, filtering, alarming, or storing via an electrical linkage from the at least one means for conducting electrical signals using at least three conductive electrodes. The electrical linkage can include any form of direct physical linkage or transmission, and can further include any form of wireless transmission.

At least one means for signal pre-processing, preamplifying, amplifying, processing, displaying, analyzing, filtering, alarming, or storing is not limited to any particular device capable of performing at least one of such function(s) and can, for example, include a wrist watch, data logger diary, personal digital assistant (PDA), exercise machine (treadmill, etc.), ECG monitor, oscilloscope, laptop, personal computer, audio-visual display unit, alarm system, or Cardiac Event Monitor.

At least one means for signal pre-processing, preamplifying, amplifying, processing, displaying, analyzing, filtering, alarming, or storing can be external or internal to the wearable item, for example, it can be housed in a device that is integrated with, or removable from, the wearable item. For example, in one embodiment it can be attached to the wearable item (such as via a mechanical fastening mechanism, such as snaps, etc.). It can also be connected to the wearable item via at least one wire or cable (which may, for example, be detachable from the wearable item). It can also be capable of wireless transmission to and from the wearable item, such as is done with hospital ECG monitors such as ECG recorders and Cardiac Event monitors.

The at least one means for signal pre-processing can include a circuit designed to reduce common-mode electrical noise that would otherwise be received from the conductive electrodes. Such a circuit can include: (i) a low-gain high common-mode rejection ratio and high input impedance first stage of amplification of electrical signals fed to the circuit from at least two conductive electrodes; (ii) a high-pass filtering stage (iii) a high-gain second stage of amplification of output from the high-pass filtering stage; (iv) a feedback stage wherein the common-mode noise signal from the first stage is buffered, amplified and inverted before being fed back to a lead to at least a third conductive electrode. The present invention can be further described with reference to the figures.

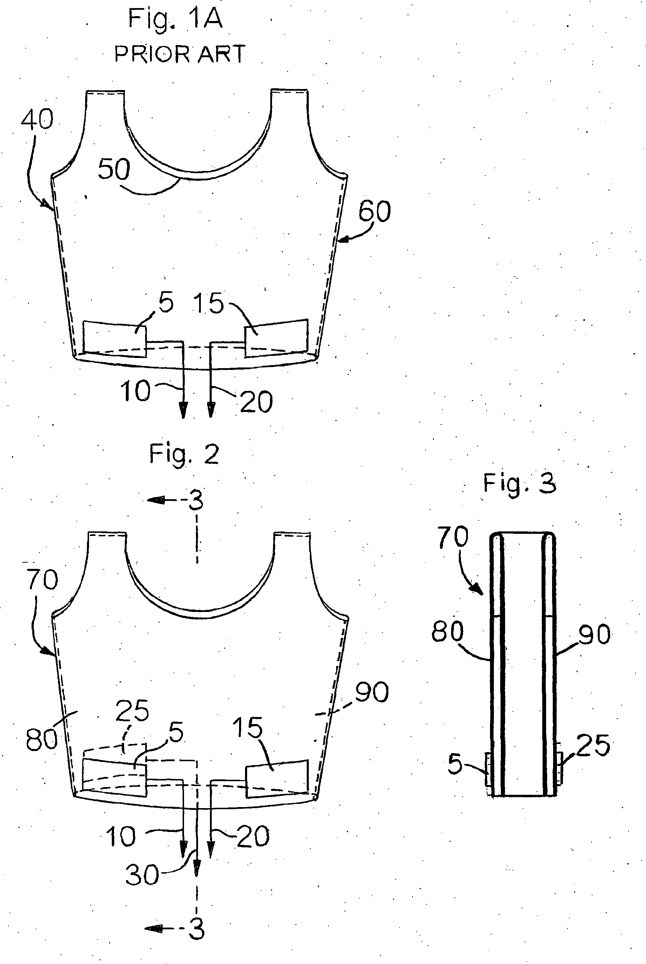

FIG. 1 shows an exemplary garment (a sports bra) 40 of the prior art having two electrodes. The garment includes an inner portion 50 and an outer portion 60 folded over and in mutual contact. Included in the bra 40 are two textile-based electrodes, 5 and 15, fully integrated with the garment 40, such as the conductive electrodes in U.S. 2006/0211934 (Pat. Appln No. 11/082,240). The textile electrodes of garment 40 incorporate conductive float yarns in mutual contact thereby providing an electrical connection between portions 50 and 60 and the skin of the wearer. The textile-based electrodes 5 and 15 are on the front of the outer portion 60 and placed low on the thorax to receive a heart signal communicated from skin contact between electrodes (not shown) of the inner portion 50 for heart rate monitoring.

FIG. 2 shows an exemplary garment (a sports bra) 70 of the invention comprising an inner portion 80 and an outer portion 90 folded over and in mutual contact. Included in the bra 70 are three textile-based electrodes 5, 15 and 25, respectively, fully integrated with the garment 70. In FIG. 2, two electrodes 5, 15 are represented on the front outer portion 90 and a third electrode 25 on the back of the garment 70. The three textile-based electrodes 5, 15 and 25 are fully integrated with the garment 70, and can be of the same construction as the textile-based electrodes in U.S. 2006/0211934 (Pat. Appln No. 11/082,240). The textile electrodes of garment 70 incorporate conductive float yarns in mutual contact thereby providing an electrical connection between portions 80 and 90 and the skin of the wearer. The conductive electrodes 5 and 15 are on the front of the outer portion 90 and the textile electrode 25 is on the back outer portion 90. The electrodes are placed low on the thorax to receive a heart signal communicated from skin-contact between electrodes (not shown) of the inner portion 80.

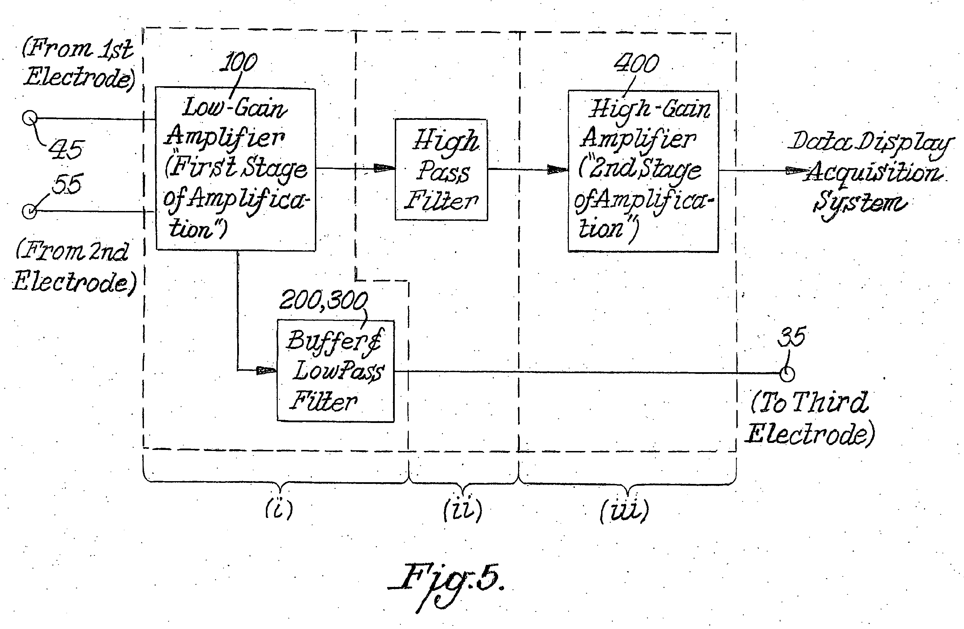

FIG. 4 schematically represents an exemplary electronic circuit for a signal pre-processor that can be used in embodiments of the invention. This signal pre-processor can accept three inputs, such as from garment 70 electrodes 5, 15 and 25 via conductive leads 10, 20 and 30 shown in FIG. 2. The leads 10 and 20 are continuous with the front electrodes 5 and 15 of garment 70. Leads 10 and 20 are used to pick up the ECG signal of the wearer introduced to the signal pre-processor circuit in FIG. 4 at 45 and 55, respectively. A third electrode 25 of garment 70 is continuous with lead 30. Lead 30 of FIG. 2 is introduced to the signal pre-processor circuit at 35 in FIG. 4. As illustrated in the block diagram of FIG. 5, the amplifier circuit includes three stages. These stages are: (i) a low-gain first stage having the differential amplifier with a high input impedance and high common-mode rejection ratio, 100, followed by a (ii) high-pass RC filter stage 160 and 180, and (iii) a secondary high-gain amplifier comprising operational amplifier 400.

The first stage of the signal pre-processor represented in FIGS. 4 and 5 can have a high input impedance and can have a voltage gain of about 20. The high-pass filter can have a cut-off frequency of about 0.5Hz. A simple RC filter (160,180) can be used for the high-pass filtering. The secondary stage of amplification, inverting operational amplifier 400, can have a voltage gain of about 100. The input to inverting operational amplifier 400 is the output of the RC filter.

In the simple case of a heart-rate monitor, the polarity of the output signal from amplifier 400 in FIG. 4 should be immaterial. Accordingly, leads 10 and 20 of FIG. 2 can be connected as convenient to inputs 45 and 55 of FIG. 2 in either configuration. In the case where an ECG quality signal is required, then lead 20 should be connected to the inverting (-) input and lead 10 should be connected to the non-inverting (+) input of the differential amplifier 100 of FIG. 4.

Output of the second stage of amplification 400 may be connected to a pulse-detection circuit or a data acquisition system for storage of the ECG or heart rate. The common-mode electrical noise from the first stage of amplification 100 can be buffered, inverted and amplified before being fed back to the third lead 35, also called the active lead. This measure can be taken to reduce the common-mode electrical interference of the system. The common-mode signal of the system can be inverted and fed back into the body via the third electrode 25 in order to improve the common-mode rejection ratio (CMRR) of the system (with a higher CMRR being better).

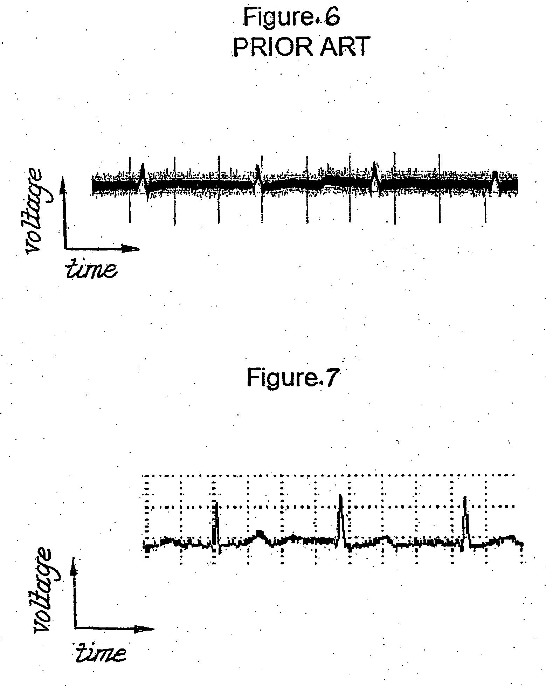

Output signals from the inverting operational amplifier 400 are shown in FIGS. 6 and 7. As shown in these figures, waveforms of the digital storage oscilloscope are much improved using three fabric electrodes (FIG. 7), where one electrode functions as an active electrode to improve the signal-to-noise-ratio CMRR of the system, as compared to a system having only two fabric electrodes (FIG. 6). These figures show screen prints of voltage versus time from a TDS1000 oscilloscope available from Tektronix, Inc., Beaverton, Oregon, USA.

The improved performance of the three electrode-based system of the invention can be at least partially explained by the ability of the system to amplify differential input signals as opposed to background noise. In this regard, in the case of the prior art two-electrode system, any noise generated by the body, such as static build up or motion artifacts, has an associated large common-mode component. Because of this, the two electrodes "see" the electrical noise at the same time and in the same way. While, such a signal presented to a theoretically "perfect" differential amplifier would not present a problem (as the amplifier output should only be a function of the difference of the two input signals at the input electrode nodes), a real world differential amplifier is not immune to common-mode noise In particular, any imperfect components and small differences among resistances in the a real world amplifier result in the amplifier turning common-mode into differential mode, albeit at a low level (although it is possible that common-mode noise can actually be larger than the signal to be measured). As a result, noise from sources such as those identified above will be amplified with the signal, and will therefore serve to reduce the signal-to-noise ratio and thus reduce the sensitivity and effectiveness of a two electrode heart rate monitor system

In comparison to the two-electrode system described above, the three-electrode system includes a third or active electrode, which can take common-mode noise, as seen by the amplifier, buffer it and invert it before feeding it back to the body. Essentially, the feedback reduces the common-mode which reduces the feedback until there is a balance of no noise and no common-mode, effectively cancelling much of the noise at the source. Through these means, the amount of common-mode electrical noise arriving at the amplifier can be much reduced. For example, the amplifier's CMMR (common-mode rejection ratio) can be about 100 db. Such a CMMR can provide an output signal which is a much better function of only the true differential between the electrodes with significantly reduced noise and a much higher quality signal, as shown in FIG. 7.

In addition, in two-electrode systems, such as the two-electrode system described above, it may be desirable to wet, for example, the fabric surface in order to enhance the surface-to-surface electrical connection from the user in order to obtain fast signal pick-up. By comparison, in the three-electrode system of the present invention, the need for such wetting can be reduced or eliminated. In other words, the three electrode system of the present invention can provide a dry textile electrode system.

The performance of two-electrode systems may also be enhanced or improved when the portion of a wearable item containing the electrodes (such as a strap or band) is cinched or tightened against the body to promote good electrical contact between the skin and the textile surface.

### EXAMPLES

### Heart Rate Monitoring Belts

The following example of the invention illustrates an embodiment in the form of heart rate monitoring belts.

The exemplified heart-rate monitoring belts include fabric systems that are essentially identical to those disclosed in the examples of U.S. Pat. Appln No. 11/082,240, filed March 16, 2005. Specifically, these heart-rate monitoring belts are made by circular knitting using a SMA-8-TOP1 seamless, 13 inch body size, knitting machine from SANTONI (from GRUPPO LONATI, Italy) (hereinafter, "the SANTONI knitting machine"). In making the heart-rate monitoring belts, a combination of different knitting constructions (including jersey and mock rib knit construction) using various types of yarns can be used.

In one example, the fabric system of the heart rate monitoring belt includes at least one electrode or conductive region within a base fabric. The at least one electrode region is made using *Xstatic*® yarns of a silver metallized nylon yarn of 70 denier and 34 filaments from Laird Sauquoit Industries (Scranton, Pennsylvania, USA 18505) (hereinafter, "*Xstatic*® 70/34"). The base fabric is a knit of Coolmax® 70/88 micro denier polyester yarn from INVISTA ("Coolmax®"), plated with Lycra® spandex (T-902C 260d). The Coolmax® and Lycra® spandex are knitted together using the SANTONI knitting machine at a ratio of about 92% Coolmax® and 8% Lycra® spandex (ratios of from about 75 to about 100% Coolmax® and from 0 to about 25% Lycra® spandex are also possible), wherein both plain jersey stitching and mock rib (1x1, 3x1, 2x1, 2x2) stitching are used in the regions of the fabric containing the conductive electrodes (the "conductive regions"), as well as the non-conductive regions of the, fabric.

For the regions of the fabric containing the conductive electrodes (or "conductive regions"), a conductive yarn is knitted on one side of the base fabric (on the non-float regions) using the SANTONI knitting machine. The conductive yarn is X-static® 70/34 (although composite yarns form Bekaert having approximately 80% polyester and 20% stainless steel could also be used). The basic construction of the conductive fabric regions is identical to that represented in U.S. 2006/0211934 (Pat. Appln No.11/082,240).

The electrodes represented as 5, 15 and 25 of FIG. 4 are electrically contacted to conductors 10, 20 and 30, respectively. These conductors are optionally constructed from traditional hard copper conductors or stretch and recovery conductive yarns of the type disclosed in U.S. Published Pat. Appln No. 2004/0237494 A1.

An amplifier circuit, such as shown in FIG. 4, is useful for acquiring the signal from a three electrode heart-rate monitor belt, or a sports bra according to the representation of FIG. 2. Table 1 lists components from which a skilled person may construct this circuit.

**Table 1.**

| Circuit Element in FIG. 4. | Description |
|---|---|
| 100 | INA326/INA3 27 Texas Instruments amplifier |
| 110 | 40 k ohm resistor |
| 120 | 40 k ohm resistor |
| 130 | 20 k ohm resistor |
| 140 | 20 k ohm resistor |
| 150 | 40 pF capacitor |
| 160 | 100 mF capacitor |
| 170 | 1 k ohm resistor |
| 180 | 3.2 k ohm resistor |
| 190 | 100 k ohm resistor |
| 200 | Low noise Op-amp |
| 300 | Low noise Op-amp |
| 400 | Low noise Op-amp |
| Note shown in FIG. 4 are power supplies: 5 Volts or 6.6 Volts dual voltage supply commonly employed in the art. | |

For comparison purposes, the *POLAR S810i* "soft" and "hard" heart-rate monitor belts are referenced. These belts are essentially identical to those disclosed in patent document WO 02/071935, assigned to RTO Holding OY. The *POLAR S810i* comparison heart-rate monitor belts incorporate just two skin contacting electrodes and conform essentially to the device represented by FIG. 1 herein.

As a test method, the quality of signal pick-up is rated by a panel of experts wearing the *POLAR S810i* and then a three-electrode belt embodiment according to the invention (as shown in FIG. 2). The signal quality of the POLAR belts is first rated for speed of first signal acquisition during the onset of a prescribed exercise routine for each wearer. The presence of electrical noise or other signal degradation in the waveform is also noted during vigorous motion or exercise by the wearer.

The output signal from a circuit of the type represented by FIG. 4 is displayed using a digital storage oscilloscope (DSO), which use is well-known for displaying or representing a heart signal (a DSO equivalent to that used in this example is the model number TDS1000 available from Tektronix, Inc., Beaverton, Oregon, USA). In particular, the output from the operational amplifier 400 in FIG. 4 is connected to a DSO vertical amplifier and the voltage output at discrete time intervals is sampled via the analog-to-digital converter in the horizontal input to the DSO. The result is a waveform such as those represented in FIGS. 6 and 7.

The improved performance of the three textile electrode is shown by the waveform corresponding to that represented in FIG. 7. This FIG. 7 signal has a higher signal-to-noise ratio than that of FIG. 6, which represents a heart-rate waveform from a prior-art heart-rate monitor belt (e.g. the *POLAR S810i*) with two skin contacting electrodes.

A sports bra or belt constructed according to the foregoing methods and materials will be expected to be capable of exerting at least 10 mm Hg pressure and more typically about 20 mm Hg pressure on the skin-contact regions of the electrodes. Generally, such a tightly fitting skin contact provides a reliable signal pick up that is sufficiently free from electrical noise induced by body movements of the wearer. In combination with three textile electrodes and the signal acquisition, amplification, and filtering circuit herein disclosed, a superior performing heart-rate monitoring system is disclosed.

The present invention is as defined by the appended claims.

## Claims

1. A wearable system for monitoring at least one physiological event or physiological characteristic of a wearer comprising:
(a) a wearable (70) comprising:
(i) at least one substantially non-electrically conductive yarn; and
(ii) three conductive electrodes, wherein a first electrode (5) and a second electrode (15) are placed on the front portion of the wearable, and wherein a third electrode (25) is placed on the back of the wearable; and further comprising (b) at least one means (10, 20, 30) for conducting from the wearable, electrical signals associated with the at least one physiological event or physiological characteristic of the wearer, wherein the means (10, 20, 30) for conducting electrical signals is electrically linked to at least one means for signal pre-processing; and
**characterized in that** the means for signal pre-processing comprises a circuit comprising:
(i) a tuned low-gain high input impedance first stage of amplification (100) of electrical signals fed to the circuit from at least two conductive electrodes (5, 15);
(ii) a high- pass filtering stage (160, 180);
(iii) a high-gain second stage of amplification (400) of output from the high-pass filtering stage (160, 180); and
(iv) a feedback stage wherein the common-mode electrical noise from the first stage is buffered, amplified and inverted before being fed back to a lead to the third conductive electrode (25) thereby increasing the common-mode rejection ratio (CMRR) of the system.

2. The wearable according to claim 1, wherein one or more of the three conductive electrodes (5, 15, 25) are incorporated into or on to the wearable (70) by at least one method selected from the group consisting of: (i) woven into and/or onto the wearable; (ii) knitted into and/or onto the wearable; (iii) printed onto the wearable; (iv) heat-transferred onto the wearable; (v), glued, laminated or sandwiched onto or between layers of the wearable; and (vi) mechanically fastened onto the wearable.

3. The wearable according to claims 1 or 2, wherein one or more of the three conductive electrodes (5, 15, 25) comprise at least one material selected from the group consisting of: (i) metal wire, (ii) conductive fiber, (iii) conductive ink, (iv) conductive polymer, (v) conductive yarn, and (vi) metal.

4. The wearable according to claim 1, wherein the at least one conductive yarn comprises at least one member of the group consisting of:
(i) a synthetic conductive polymer;
(ii) a synthetic polymer coated by a metal;
(iii) a synthetic polymer composition comprising metal particles or filaments; and
(iv) a synthetic polymer twisted or wrapped with a metal wire.

5. The wearable according to claim 1 , wherein the means for conducting electrical signals comprises at least one conductive surface or region having direct contact with a wearer's skin, wherein said means for conducting electrical signals is capable of electrically linking the at least one conductive surface or region to at least one means for signal pre-processing, preamplifying, amplifying, processing, displaying, analyzing, filtering, alarming, and/or storing said at least one physiological event or physiological characteristic.

6. The wearable according to claim 5, wherein the means for conducting electrical signals comprises at least one interconnect device or material.

7. The wearable according to claim 6, wherein the interconnect device is a conductive interconnect bridge selected from the group consisting of: (i) a snap; (ii) a conductive thread; (iii) a conductive wire; (iv) touching interior conductive floats;(v) metal grommets; (vi) conductive glue or hot melt material; and (vii) fuzzy inner surface brush contacts.

8. The wearable according to claim 1, wherein the means for signal pre-processing is housed in at least one device that is integrated with or removable from the wearable.

9. The wearable according to claim 1, wherein the means (10, 20, 30) for conducting electrical signals is electrically linked to the device via wireless transmission.

10. The wearable according to claim 8, wherein the at least one device is selected from the group consisting of: (i) a wrist watch; (ii) a data logger diary; (iii) a personal digital assistant (PDA); (iv) an exercise machine; (v) an electrocardiogram (ECG) monitor; (vi) an oscilloscope; (vii) a laptop or personal computer; (viii) an audio-visual display unit; (ix) an alarm system; (x) a Cardiac Event Monitor; and (xi) a Pacemaker.

11. The wearable according to claim 1 or 10, wherein the physiological event or physiological characteristic comprises at least one event or characteristic selected form the group consisting of electrocardiogram (ECG) or heart rate, breathing rate, electroencephalogram (EEG), electromyogram (EMG), and Electro Gastrogram (EGG).

12. The wearable of claim 1, wherein the electrode skin impedances of the three electrodes are the same.

13. The wearable of claim 1 in the form of a garment.

14. The wearable of claim 13, wherein the garment is selected from the group consisting of a bra, a shirt, an undergarment, a vest, a bodysuit, a sock, a glove, a stocking, a belt, a band, a strap and a jacket.

15. The wearable of claim 14, wherein the band is selected from the group consisting of a torso band, a waist band, an arm band, a leg band, a neck band, and a wrist band.

## Patentansprüche

1. Ein Wearable-System zum Beobachten zumindest eines physiologischen Ereignisses oder einer physiologischen Charakteristik eines Trägers aufweisend:
(a) ein Wearable (70) aufweisend:
(i) zumindest ein im Wesentlichen nicht elektrisch leitfähiges Garn; und
(ii) drei leitfähige Elektroden, wobei eine erste Elektrode (5) und eine zweite Elektrode (15) auf dem vorderen Teil des Wearables platziert sind, und wobei eine dritte Elektrode (25) auf der Rückseite des Wearable platziert ist; und weiter aufweisend
(b) zumindest ein Mittel (10, 20, 30) zum Ableiten elektrischer Signale von dem Wearable, welche mit dem zumindest einen physiologischen Ereignis oder physiologischen Charakteristik des Trägers verknüpft sind, wobei die Mittel (10, 20, 30) zum Ableiten elektrischer Signale mit zumindest einem Mittel zur Signalvorverarbeitung elektrisch verbunden ist; und **dadurch gekennzeichnet, dass** das Mittel zur Signalvorverarbeitung einen Schaltkreis aufweist, welcher aufweist:
(i) eine abgestimmte schwach-verstärkende ein hohe Eingangsimpedanz aufweisende erste Verstärkerstufe (100) von elektrischen Signalen welche dem Schaltkreis von zumindest zwei leitfähigen Elektroden (5, 5) zugeführt werden;
(ii) eine Hochpassfilterstufe (160, 180);
(iii) eine hoch-verstärkende zweite Verstärkerstufe (400) des Ausgangs der Hochpassfilterstufe (160, 80); und
(iv) eine Rückkopplungsstufe, wobei das elektrische Gleichtaktrauschen von der ersten Stufe gepuffert, verstärkt und invertiert wird bevor es an eine Leitung der dritten leitfähigen Elektrode (25) zurückgeführt wird, wodurch das Gleichtaktunterdrückungsverhältnis (common mode rejection ratio, CMRR) des Systems vergrößert wird.

2. Wearable nach Anspruch 1, wobei eine oder mehrere der drei leitfähigen Elektroden (5, 15, 25) in das Wearable (70) eingebracht oder an dem Wearable (70) angebracht ist durch zumindest ein Verfahren aus der Gruppe bestehend aus: (i) eingewebt in und/oder auf das Wearable; (ii) eingestrickt oder eingewirkt in und/oder auf das Wearable; (iii) aufgedruckt auf das Wearable; (iv) durch Hitze auf das Wearable übertragen; (v) geklebt, laminiert oder eingelegt in oder zwischen Schichten des Wearables; und (vi) mechanisch auf dem Wearable befestigt ist.

3. Wearable nach Ansprüchen 1 oder 2, wobei eine oder mehrere der drei leitfähigen Elektroden (5, 15, 25) zumindest ein Material aufweist, welches ausgewählt wurde aus der Gruppe bestehend aus: (i) Metalldraht, (ii) leitfähige Faser, (iii) leitfähige Tinte, (iv) leitfähiges Polymer, (v) leitfähiges Garn, und (vi) Metall.

4. Wearable nach Anspruch 1, wobei das zumindest eine leitfähige Garn zumindest einen Bestandteil aufweist aus der Gruppe bestehend aus: (i) ein synthetisches leitfähiges Polymer; (ii) ein synthetisches mit einem Metall beschichtetes Polymer; (iii) eine synthetische Polymerzusammensetzung, welche Metallpartikel oder Metallfilamente aufweist; und (iv) ein synthetisches Polymer, welches mit einem Metalldraht verdrillt oder mit diesem umwickelt ist.

5. Wearable nach Anspruch 1, wobei das Mittel zum Ableiten elektrischer Signale zumindest eine leitfähige Oberfläche oder Bereich aufweist, welche(r) direkte Berührung mit der Haut eines Trägers hat, wobei das Mittel zum Ableiten elektrischer Signale geeignet ist, die zumindest eine leitfähige Oberfläche oder Bereich mit zumindest einem Mittel zur Signalvorverarbeitung, Vorverstärkung, Verstärkung, Verarbeitung, Anzeige, Analysieren, Filtern, Alarmgeben, und/oder Speichern des zumindest einen physiologischen Ereignisses oder physiologischen Charakteristik elektrisch zu verbinden.

6. Wearable nach Anspruch 5, wobei das Mittel zum Ableiten elektrischer Signale zumindest ein Verbindungsgerät oder Material aufweist.

7. Wearable nach Anspruch 6, wobei das Verbindungsgerät eine leitfähige Verbindungsbrücke ist, ausgewählt aus der Gruppe bestehend aus: (i) eine Schnappverbindung; (ii) ein leitfähiger Faden; (iii) ein leitfähiger Draht; (iv) sich berührende innere leitfähige Schwimmkörper; (v) Metallösen; (vi) leitfähiger Klebstoff oder Schmelzklebermaterial; und (vii) flaumige innere Oberflächenbürstenkontakte.

8. Wearable nach Anspruch 1, wobei das Mittel zur Signalvorverarbeitung in zumindest einem Gerät untergebracht ist, welches in oder lösbar von dem Wearable integriert ist.

9. Wearable nach Anspruch 1, wobei das Mittel (10, 20, 30) zum Ableiten elektrischer Signale mit dem Gerät über eine drahtlose Übertragung elektrisch verbunden ist.

10. Wearable nach Anspruch 8, wobei das zumindest eine Gerät ausgewählt ist aus der Gruppe bestehend aus: (i) eine Armbanduhr; (ii) ein Datenaufzeichnungstagebuch; (iii) ein Minicomputer (personal digital assistant, PDA); (iv) eine Trainingsmaschine; (v) ein Elektrocardiogramm- (ECG-) Monitor; (vi) ein Oszilloskop; (vii) ein Laptop oder Personal-Computer; (viii) eine audiovisuelle Anzeigeeinheit; (ix) ein Alarmsystem; (x) ein Herzereignismonitor; (xi) ein Herzschrittmacher.

11. Wearable nach Anspruch 1 oder 10, wobei das physiologische Ereignis oder die physiologische Charakteristik zumindest ein Ereignis oder Charakteristik aufweist ausgewählt aus der Gruppe bestehend aus Elektrocardiogramm (ECG) oder Herzfrequenz, Atemfrequenz, Elektroenzephalogramm (EEG), Elektromyogramm (EMG), und Elektrogastrogramm (EGG).

12. Wearable von Anspruch 1, wobei die Elektrodenhautimpedanzen der drei Elektroden die gleichen sind.

13. Wearable nach Anspruch 1 in der Form eines Bekleidungsstücks.

14. Wearable von Anspruch 13, wobei das Bekleidungsstück ausgewählt ist aus der Gruppe bestehend aus einem Büstenhalter, einem Hemd, einer Unterwäsche, einer Weste, eines Bodys, einer Socke, eines Handschuhs, eines Strumpfs, eines Gürtels, eines Bands, eines Riemens, und einer Jacke.

15. Wearable von Anspruch 14, wobei das Band ausgewählt ist aus der Gruppe bestehend aus einem Rumpfband, einem Taillenband, einem Armband, einem Beinband, einem Nackenband, einem Handgelenksband.

## Revendications

1. Un système d'article à porter sur soi pour surveiller au moins un évènement physiologique ou une caractéristique physiologique d'un porteur, comprenant :
(a) un article à porter comprenant :
(i) au moins un fil textile substantiellement non électriquement conducteur, et
(ii) trois électrodes conductrices, une première électrode (5) et une seconde électrode (15) étant placées sur la partie avant de l'article à porter, et une troisième électrode (25) étant placée au dos de l'article à porter ; et
comprenant en outre :
(b) au moins un moyen (10, 20, 30) pour conduire à partir de l'article à porter des signaux électriques associés à l'au moins un événement physiologique ou caractéristique physiologique du porteur, le moyen (10, 20, 30) pour conduire des signaux électriques étant électriquement relié à au moins un moyen de prétraitement de signal ; et
**caractérisé en ce que** le moyen de prétraitement de signal comprend un circuit comportant :
(i) un premier étage accordé à haute impédance d'entrée et faible gain pour l'amplification (100) de signaux électriques appliqués au circuit depuis au moins deux électrodes conductrices (5, 5) ;
(ii) un étage de filtrage passe-haut (160, 180) ;
(iii) un second étage à gain élevé pour l'amplification (400) d'une sortie de l'étage de filtrage passe-haut (160, 80) ; et
(iv) un étage de rétroaction, le courant électrique en mode commun en provenance du premier étage étant tamponné, amplifié et inversé avant d'être renvoyé vers un conducteur électrique vers la troisième électrode conductrice (25), de manière à augmenter ainsi le taux de réjection en mode commun (CMRR) du système.

2. L'article à porter de la revendication 1, dans lequel une ou plusieurs des trois électrodes conductrices (5, 15, 25) est incorporée à l'intérieur ou sur l'article à porter (70) par au moins un procédé choisi dans le groupe formé par : (i) tissage dans et/ou sur l'article à porter ; (ii) tricotage dans et/ou sur l'article à porter ; (iii) impression sur l'article à porter ; (iv) transfert thermique sur l'article à porter ; (v) collage, stratification ou prise en sandwich sur ou entre des couches de l'article à porter ; et (vi) fixation mécanique sur l'article à porter.

3. L'article à porter des revendications 1 ou 2, dans lequel une ou plusieurs des trois électrodes conductrices (5, 15, 25) comprend au moins un matériau choisi dans le groupe formé par : (i) fil électrique métallique, (ii) fibre conductrice, (iii) encre conductrice, (iv) polymère conducteur, (v) fil textile conducteur, et (vi) métal.

4. L'article à porter de la revendication 1, dans lequel le au moins un fil textile conducteur comprend au moins un membre du groupe formé par : (i) un polymère conducteur synthétique, (ii) un polymère synthétique revêtu d'un métal, (iii) une composition de polymère synthétique comprenant des filaments ou particules métalliques ; et (iv) un polymère synthétique torsadé ou enroulé par un fil électrique métallique.

5. L'article à porter de la revendication 1, dans lequel le moyen pour conduire les signaux électriques comprend au moins une région ou surface conductrice en contact direct avec une peau du porteur, ledit moyen pour conduire des signaux électriques étant capable de relier électriquement la au moins une région ou surface conductrice à au moins un moyen de prétraitement de signal, de préamplification, d'amplification, de traitement, d'affichage, d'analyse, de filtrage, d'alarme et/ou de mémorisation dudit au moins un événement physiologique ou caractéristique physiologique.

6. L'article à porter de la revendication 5, dans lequel le moyen pour conduire les signaux électriques comprend au moins un matériau ou dispositif d'interconnexion.

7. L'article à porter de la revendication 6, dans lequel le dispositif d'interconnexion est un pont d'interconnexion conducteur choisi dans le groupe formé par : (i) un bouton-pression ; (ii) un fil textile conducteur ; (iii) un fil électrique conducteur ; (iv) des flottants conducteurs intérieurs venant en contact ; (v) des oeillets métalliques ; (vi) une matière thermofusible ou une colle conductrice ; et (vii) des contacts en balai à surface interne irrégulière.

8. L'article à porter de la revendication 1, dans lequel le moyen de prétraitement de signal est logé dans au moins un dispositif qui est intégré à l'article à porter ou séparable de celui-ci.

9. L'article à porter de la revendication 1, dans lequel le moyen (10, 20, 30) pour conduire les signaux électriques est électriquement relié au dispositif par transmission sans fil.

10. L'article à porter de la revendication 8, dans lequel le au moins un dispositif est choisi dans le groupe formé par : (i) une montre-bracelet ; (ii) un journal d'enregistrement de données ; (iii) un assistant numérique personnel (PDA) ; (iv) une machine d'entrainement ; (v) un moniteur d'électrocardiogramme (ECG) ; (vi) un oscilloscope ; (vii) un ordinateur personnel ou ordinateur portable ; (viii) une unité d'affichage audiovisuelle ; (ix) un système d'alarme ; (x) un moniteur d'événements cardiaques ; et (xii) un stimulation cardiaque.

11. L'article à porter de la revendication 1 ou 10, dans lequel l'événement physiologique ou caractéristique physiologique comprend au moins un événement ou caractéristique choisi dans le groupe formé par : électrocardiogramme (ECG) ou fréquence cardiaque, fréquence respiratoire, électroencéphalogramme (EEG), électromyogramme (EMG), et électrogastrogramme (EGG).

12. L'article à porter de la revendication 1, dans lequel les impédances de peau d'électrode des trois électrodes sont les mêmes.

13. L'article à porter de la revendication 1, sous forme d'un vêtement.

14. L'article à porter de la revendication 13, dans lequel le vêtement est choisi dans le groupe formé par : un soutien-gorge, une chemise, un sous-vêtement, un gilet, une combinaison, une chaussette, un gant, un bas, une ceinture, un bandage, une lanière et une veste.

15. L'article à porter de la revendication 14, dans lequel le bandage est choisi dans le groupe formé par un bandage de torse, un bandage de poitrine, un bandage de bras, un bandage de jambe, un bandage de cou et un bandage de poignet.
